(19) Europäisches Patentamt
European Patent Office
Office européen des brevets

(11) Veröffentlichungsnummer: **0 201 055 B1**

(12) **EUROPÄISCHE PATENTSCHRIFT**

(45) Veröffentlichungstag der Patentschrift: **15.05.91**

(51) Int. Cl.⁵: **C07C 211/63**, C07C 309/03, C07D 213/20

(21) Anmeldenummer: **86106025.9**

(22) Anmeldetag: **02.05.86**

# (54) Verfahren zur Herstellung von halogenidfreien quaternären Ammoniumsalzen.

(30) Priorität: **10.05.85 DE 3516843**

(43) Veröffentlichungstag der Anmeldung: **12.11.86 Patentblatt 86/46**

(45) Bekanntmachung des Hinweises auf die Patenterteilung: **15.05.91 Patentblatt 91/20**

(84) Benannte Vertragsstaaten: **DE**

(56) Entgegenhaltungen: **DE-C- 883 437**

(73) Patentinhaber: **HOECHST AKTIENGESELLSCHAFT**
**Postfach 80 03 20**
**W-6230 Frankfurt am Main 80(DE)**

(72) Erfinder: **Wojtech, Bernhard, Dr.**
**Kelkheimer Strasse 67**
**W-6232 Bad Soden am Taunus(DE)**
Erfinder: **Interthal, Werner, Dr.**
**Dr Ludwig-Opel-Strasse 62**
**W-6090 Rüsselsheim(DE)**
Erfinder: **Ohlendorf, Dieter, Dr.**
**Am Kühlen Grund 4**
**W-6237 Liederbach(DE)**

**EP 0 201 055 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Stoffe, die den Reibungswiderstand strömender Flüssigkeiten verringern, so daß diese unter sonst gleichen Bedingungen schneller strömen, werden Strömungsbeschleuniger (engl. drag reducing agents) genannt. Diese Wirkung zeigen einige hochmolekulare Stoffe (Polyethylenoxide, Polyacrylamide) oder die Lösungen einiger Tenside. Wegen ihres irreversiblen Abbaus bei hoher Scher- und Dehnbeanspruchung sind die hochmolekularen Substanzen als Zusätze für geschlossene Wasserkreisläufe, z.B. Kühlkreisläufe und Fernwärmenetze, nicht brauchbar. Diesen Nachteil weisen Tenside, die als Strömungsbeschleuniger wirksamen sind, z.B. die Alkalisalze höherer Fettsäuren, oder kationische Tenside, wie z.B. Cetylpyridiniumbromid, nicht auf. Der Hauptnachteil alle bisher bekannten Tensidlösungen ist aber, daß sie oberhalb 90 °C ihre Wirksamkeit als Strömungsbeschleuniger verlieren und deshalb für Fernwärmenetze völlig ungeeignet sind. Aus EP-A-97 926 ist die neue Klasse mizellenbildender Tenside der Formel I wie nachstehend angegeben bekannt bei der es gefunden wurde, daß sie schon in kleinsten Konzentrationen wirksam sind und auch bei Temperaturen oberhalb 90 °C bei Dauerbeanspruchung effektiv bleiben. Es handelt sich dabei um Salze, die als Kation Alkyltrimethylammonium-oder Alkylpyridinium-Ionen enthalten. Die jeweiligen Anionen leiten sich ab von aromatischen Carbon- und Sulfonsäuren. Voraussetzung für die Verwendung dieser Tensidsalze als Strömungsbeschleuniger besonders in geschlossenen Systemen ist aber, daß sie keine Korrosionswirkung haben. Dies bedeutet, daß diese Verbindungen frei von Halogenid-Ionen hergestellt werden.

Für die halogenidfreie Herstellung dieser neuen quaternären Ammoniumsalze sind folgende Verfahren bekannt und beschrieben. Man löst zunächst Alkyl-trimethylammonium-halogenide bzw. Pyridinium-chloride, -bromide oder auch -jodide in wasserfreien Lösungsmitteln wie z.B. Methanol auf und gibt frisch gefälltes, zuletzt mit Methanol gewaschenes Silberhydroxid in leichtem Überschuß hinzu. Es soll möglichst kein Wasser in das System eingebracht werden, da man sonst Schwierigkeiten mit der Kristallisation bekommt. Bei kurzem Anwärmen auf ca. 50 °C vollzieht sich die Bildung des Alkyl-trimethylammonium- bzw. Pyridiniumhydroxids, das in Methanol gelöst bleibt. Bei dieser Umsetzung verschwindet die braune Farbe des Silberhydroxids weitgehend und der resultierende Niederschlag nimmt die Farbe des Silberhalogenids an. Der Silberhalogenid-Niederschlag wird dann bei ca. 15 °C abgesaugt. Das im methanolischen Filtrat vorliegende Alkyltrimethylammonium- bzw. Pyridiniumhydroxid kann durch Zugabe der stöchiometrischen Menge einer Carbonsäure, Sulfosäure oder anorganischen Säure neutralisiert werden. Durch Eindampfen des Methanols wird das gewünschte Alkyl-trimethylammoniumsalz bzw. Pyridiniumsalz gewonnen.

Bei einer Verfahrensvarinate werden zunächst die Silbersalze der betreffenden Carbonsäure hergestellt, die häufig wenig wasserlöslich sind. Man kann hier von Silberhydroxid oder Silbercarbonat ausgehen und diese mit der gewünschten Carbonsäure neutralisieren. Man kann aber auch die Alkalicarboxylate in Wasser lösen und Silbernitrat-Lösung zugeben, wenn das Silbercarboxylat ausfällt. Man saugt ab, mischt und trocknet das Silbercarboxylat. Mann kann dieses dann in der stöchiometrisch erforderlichen Menge zu einer Lösung des Alkyl-trimethylammonium- bzw. Pyridiniumhalogenids in einem wasserfreien Lösungsmittel wie Methanol geben und kurz auf 50 bis 60 °C erwärmen. Auch hier wird hinterher bei 15 °C das Silberhalogenid abgesaugt und durch Eindampfen des Filtrats das gemischte Alkyl-trimethylammonium- bzw. Pyridiniumcarboxylat gewonnen. Eine weitere Reindarstellung kann durch Umkristallisieren aus prakt. wasserfreien Lösungsmitteln (Essigester, Aceton, Acetonitril, Dichlorethan) erreicht werden.

Als weitere Möglichkeit ist auch die Gewinnung der Alkyltrimethylammonium-bzw. Pyridiniumhydroxid-Lösung durch Behandlung der Alkyl-trimethylammonium- bzw. Pyridiniumhalogenide (oder anderer Salze) mit einem stark basischen Anionenaustauscher zu erwähnen, was ebenfalls in einem wasserfreien Lösungsmittel wie z.B. Methanol geschehen muß. Daran schließt sich wiederum die Neutralisation mit der gewünschten Carbonsäure an. Alle diese Verfahren sind sehr aufwendig, unwirtschaftlich und für technische Erfordernisse ungeeignet.

Aus der DE-C-883 437 ist die Herstellung von quaternären Ammonium verbindungen über Austausch reaktionen bekannt.

Es wurde nun ein einfaches Verfahren gefunden, nach dem man die Tensidsalze der genannten Art frei von Halogen-Ionen herstellen kann.

Gegenstand der Erfindung ist ein Verfahren zur Herstellung von halogenidfreien quartaren Ammoniumsalzen der Formel I wie nachstehend angegeben, indem man eine wäßrige Lösung, die Alkalihydroxid und Tetraalkylammonium-oder Alkylpyridinium-halogenide enthält, vermischt mit einer Lösung von organischen Carbon- oder Sulfonsäuren in einem mit Wasser nicht mischbaren Lösemittel.

Bei diesem Verfahren hat jede Phase zwei Aufgaben.

Die organische Phase ist einmal Trägerphase für die hydrophobe Komponente (saure Komponente), die den Anionenteil in das Ionenpaar einbringt, und zum anderen die Aufnehmerphase für das gebildete

quaternäre Ammoniumsalz. Das Wasser ist einmal Trägerphase für das wasserlösliche quaternäre Ammoniumhalogenid, das den Kationenteil in das Ionenpaar einbringt sowie für das Alkalihydroxid, das die Kondensation bewirkt und zweitens die Aufnehmerphase für das gebildete wasserlösliche Alkalihalogenid. Die Reaktionsgleichung lautet (X= Halogenid),

$$\{\text{Kation}^{+}\text{-X}^{-} + \text{Alkali}^{+}\text{-OH}\}\ \text{wäßrige Phase} + \{\text{Anion}^{-}\text{-H}^{+}\}\ \text{organische Phase} \xrightarrow{\text{Kondensation}} \{\text{Alkali}^{+}\text{-X}^{-} + H_2O\}\ \text{wäßrige Phase} + \{\text{Kation}^{+}\text{-Anion}^{-}\}\ \text{organische Phase}$$

Die herzustellenden quartären Ammoniumsalze haben die Formel I

$$R_1\text{-}K^{\oplus}\ A^{\ominus} \qquad (I)$$

wobei $R_1$ $C_{12}$-$C_{26}$-Alkyl oder $C_{12}$-$C_{26}$-Alkenyl, $K^{\oplus}$ eine Gruppe der Formeln

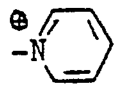

oder - $^{\oplus}N(R_2)_3$, $R_2$ $C_1$-$C_3$-Alkyl, und $A^{\ominus}$ ein Anion der folgenden Formeln bedeutet: $R_3COO^{\ominus}$ oder $R_3SO_3^{\ominus}$, wo $R_3$ $C_6$-$C_9$-Alkyl oder $C_6$-$C_9$-Alkenyl ist und die Summe der C-Atomen in $R_1$ und $R_3$ mindestens 21 betragen soll;

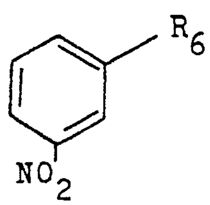 ; 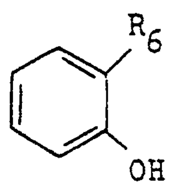 ; 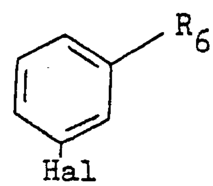 ; 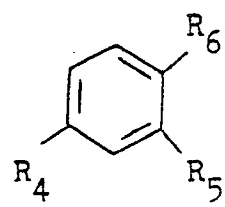 oder

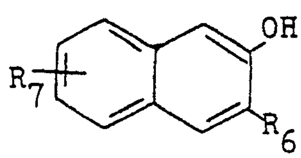

wo Hal Fluor Chlor, Brom oder Jod $R_4$ $C_1$-$C_5$Alkyl, $C_2$-$C_5$-Alkenyl oder $C_1$-$C_5$-Alkoxy in den Stellungen 3, 4, 5 und 6, $R_5$ Wasserstoff oder Hydroxy in den Stellungen 2 und 3 $R_6$ $COO^-$ oder $SO_3^-$ und $R_7$ Wasserstoff oder Methyl ist.

Ausgangsverbindungen zur Herstellung der genannten quartären Ammoniumsalze sind zum einen die Tetraalkylammoniumhalogenide und Alkylpyridiniumhalogenide der Formeln IIa und IIb

$R_1\text{-}^{+}N(R_2)_3\ Hal^{-}$ (IIa); 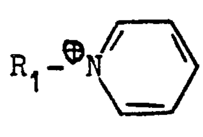 $Hal^{-}$ (IIb)

und zum anderen die den Anionen $A^-$ in der Formel I entsprechenden freien organischen Carbonsäuren und Sulfonsäuren. Für das erfindungsgemäße Verfahren werden diese Carbon- und Sulfonsäuren in einem

organischen Lösungsmittel gelöst. Die Konzentration der Säuren im organischen Lösemittel sollte zwischen 5 und 50 Gew.-%, vorzugsweise zwischen 10 und 30 Gew.-% liegen. Um diese Konzentrationen zu erreichen, kann es notwendig sein, die Lösung zu erwärmen. Als organische Lösemittel kommen alle gegenüber den Reaktionskomponenten inerten Lösemittel in Frage, in denen die obengenannten Säuren löslich sind und die gegenüber Wasser eine Mischungslücke besitzen, wie z.B. Kohlenwasserstoffe, Halogenkohlenwasserstoffe, Ether, höhere Alkohole oder Ketone beispielsweise Methylisobutylketon. Ihr Siedepunkt muß über 90 °C liegen.

Die quartären Ammoniumhalogenide werden in Wasser gelöst und zwar in einer Konzentration von 10 bis 60, vorzugsweise 20 bis 40 Gew.-%. Das die Kondensation auslösende Alkalihydroxid, z.B. Natronlauge, wird in gleicher molarer Menge wie die das Anion- bzw. Kation-tragende Verbindung ebenfalls in der wäßrigen Phase vorgelegt. Das kann durch Auflösen der festen Alkalihydroxide geschehen oder durch Zugabe einer entsprechenden Menge an Hydroxid in wäßriger Lösung.

Eine Variante des erfindungsgemäßen Verfahrens besteht darin, anstelle der Säure gleich ihre Alkalisalze zu nehmen. Dann erübrigt sich die Alkalizugabe. Die Alkalisalze können je nach ihrer Löslichkeit in das organische Lösemittel oder zusammen mit dem Ammoniumhalogeniden in die wäßrige Phase eingebracht werden. Im letztgenannten Fall besteht die organische Phase nur aus den reinen Lösemitteln.

Das Mengenverhältnis von organischer Phase zu wäßriger Phase beträgt im Durchschnitt 0,5 : 1 bis 2 : 1. Das Verhältnis kann sich aber bei begrenzter Löslichkeit der einen oder anderen Komponente in beide Richtungen verändern. In jedem Fall ist es notwendig, daß alle Komponenten in dem jeweiligen Lösemittel vollständig gelöst sind. Außerdem müssen alle Komponenten im stöchiometrischen Verhältnis vorliegen, um eine vollständige Umsetzung zu erreichen.

Für die Reaktion wird die organische Lösung gegebenenfalls bei erhöhter Temperatur, vorgelegt und die wäßrige Lösung unter kräftiger Rührung zugegeben. Die Phasen werden etwa 20 - 30 min unter Erwärmen intensiv gemischt, dann wird die Phasentrennung abgewartet und anschließend die beiden Phasen getrennt. Handelt es sich bei dem quaternären Ammoniumsalz um eine im organischen Lösemittel bei tieferen Temperaturen weniger lösliche Verbindung, muß beim Absitzen die Temperatur eingestellt werden, bei der das quaternäre Ammoniumsalz noch in Lösung bleibt.

Nach der Reaktion wird die wäßrige Phase, die praktisch nur das Alkalihalogenid enthält, verworfen. Sollten noch Anteile vom quaternären Ammoniumsalz in der wäßrigen Phase vorhanden sein, können diese durch eine Wäsche mit dem organischen Lösemittel herausgeholt werden. Die organische Phase mit dem quaternären Ammoniumsalz als Kondensationsprodukt, die meistens noch Restanteile vom Alkalihalogenid enthält, wird von diesen Verunreinigungen durch eine extraktive Wasserwäsche befreit. Das kann durch mehrmaliges Auswaschen mit Wasser geschehen (Kreuzstromextraktion) oder durch mehrstufige Gegenstromextraktion. Die Extraktionsparameter (Phasenmengenverhältnis, Stufenzahl) richten sich nach dem Restgehalt. Wegen der besseren Phasenkoaleszenz können statt reinem Wasser sehr verdünnte wäßrige Lösungen von Alkalisalzen mit einem nicht korrosiv wirkenden Anion, z.B. $NaHCO_3$, verwendet werden.

Nach der Reinigung des quaternären Ammoniumsalzes wird das organische Lösemittel durch Abdestillieren, gegebenenfalls im Vakuum, entfernt. Produkte, die bei tiefer Temperatur in organischen Lösemittel wenig oder nicht löslich sind, können alternativ durch Abkühlen ausgefällt und auf diese Weise isoliert werden. Das Filtrat wird dann direkt in den Prozeß zurückgeführt.

Das erfindungsgemäße Verfahren ermöglicht auf einfache Weise die Herstellung von Tensidsalzen frei von Halogenid-Ionen. Dabei werden durch das Auswaschen der organischen Phase mit Wasser auch geringe Restmengen an Alkalihalogenid noch zuverlässig entfernt.

Die Erfindung soll anhand folgender Beispiele erläutert werden.

Beispiel 1

Herstellung von Hexadecyltrimethylammonium-β-hydroxynaphtoat (HABON)

Als Ausgangslösungen werden eine wäßrige alkalische Hexadecyltrimethylammoniumchlorid-Lösung und eine Lösung von 2-Naphthol-3-carbonsäure (β-Hydroxynaphthoesäure) in Methylisobutylketon (MIBK) hergestellt.

216 g Ätznatron werden in 890 g Wasser zu einer 6 n Natronlauge gelöst und diese Natronlauge unter kräftigem Rühren in 5783 g einer wäßrigen Hexadecyltrimethylammoniumchlorid-Lösung (29,11 %ig) geschüttet und homogenisiert.

1000 g 2-Naphthol-3-carbonsäure (99 %ig) werden in 5550 g MIBK bei 80 - 85 °C unter Rühren

vollständig in Lösung gebracht. In diese 80° C heiße Carbonsäure-Lösung wird dann unter kräftigem Rühren die gesamte alkalische quaternäre Ammoniumchlorid-Lösung geschüttet. Das Reaktionsgemisch bleibt bei etwa 50 - 60° C weitere 20 Minuten unter kräftiger Rührung (gute Phasendurchmischung erforderlich). Dann wartet man ebenfalls bei etwa 50 - 60° C die vollständige Phasentrennung ab. Die wäßrige NaCl-haltige Lösung wird verworfen. Die MIBK-Lösung, die 99,3 % des gebildeten HABON und 28,4 % des gebildeten NaCl enthält, muß bis zur Einspeisung in den Extraktor wegen des NaCl-Gehaltes weiterhin auf ca. 50° C gehalten werden.

Durch Wasserwäsche im Volumenverhältnis MIBK-Lösung/Wasser = 2/1 wird in einem 6-stufigen Extraktor das NaCl aus der MIBK-Lösung jetzt bei Raumtemperatur herausgewaschen. Dabei werden 99,99 % des NaCl entfernt. Der HABON-Verlust im Waschwasser beträgt 0,5 %. Im Vakuumverdampfer wird aus dem Extrakt das MIBK bei Temperaturen unter 50° C abgedampft und so die Reinsubstanz gewonnen. Ausbeute 2448 g HABON.

Beispiel 2

Herstellung von $C_{20}$-$C_{22}$-Alkyl-trimethylammonium-β-hydroxynaphthoat (DOBON)

Ausgangslösungen sind eine wäßrige alkalische Docosyl-(Eicosyl)-trimethylammoniumchlorid-Lösung und eine 2-Naphthol-3-carbonsäure-Lösung in Methylisobutylketon (MIBK).

2390 g $C_{20}$-$C_{22}$-Alkyl-trimethylammoniumchlorid (technische Ware, feucht, Gehalt an Aktivsubstanz 80 % ± 2 %) werden in 9600 g $H_2O$ bei 80° C gelöst und auf dieser Temperatur gehalten. Man setzt nun unter kräftigem Rühren eine 6 n Natronlauge zu, die vorher aus 216 g Ätznatron und 890 g Wasser hergestellt wurde. Diese alkalische quternäre Ammoniumchlorid-Lösung muß ebenfalls auf 80° C gehalten werden.

1000 g 2-Naphthol-3-carbonsäure werden in 6600 g MIBK bei 80 - 85° C unter Rühren vollständig in Lösung gebracht und die Lösung auf 80° C gehalten. In die 80° C heiße Carbonsäure-Lösung wird unter kräftigem Rühren die gesamte alkalische quaternäre Ammoniumchlorid-Lösung geschüttet. Das Reaktionsgemisch bleibt bei etwa 80° C weitere 20 Minuten unter kräftiger Rührung (gute Phasenmischung erforderlich). Dann wartet man ebenfalls bei 70 - 80° C die vollständige Phasentrennung ab.

Die wäßrige NaCl-haltige Phase wird verworfen. Die MIBK-Phase, die 99 % des gebildeten DOBON und 10 % des gebildeten NaCl enthält, muß zur Entfernung des NaCl-Restes einer Wasserwäsche unterworfen werden. Um Ausfällungen zu verhindern, wird die Lösung auf Temperaturen zwischen 70° C und 80° C gehalten.

Zur Wasserwäsche verwendet man wegen der besseren Koaleszenzwirkung eine 0,5 %ige $NaHCO_3$-Lösung. Die extraktive Wäsche wird in einem 6-stufigen Extraktor bei 80° C und einem Volumenverhältnis Speiselösung/Waschflüssigkeit = 2/1 ausgeführt. Der DOBON-Verlust im Waschwasser beträgt 1 Gew.-%. Der NaCl-Gehalt in der MIBK-Phase wird bei der Wäsche um über 98,4 % von 3,1 mg NaCl/g auf < 0,05 mg/g reduziert.

Zur Gewinnung des DOBON aus der heißen MIBK-Lösung kann das MIBK im Vakuumverdampfer destillativ abgetrennt werden. Ohne Destillation lassen sich etwa 96 % des DOBON durch Ausfällung gewinnen, wenn die MIBK-Lösung auf Raumtemperatur abgekühlt wird. Das ausgefallene DOBON wird abfiltriert und das Filtrat anschließend zur Gewinnung der Restmenge eingedampft. Das MIBK-Filtrat mit der Restmenge an DOBON kann direkt als Vorlage für einen neuen Ansatz verwendet werden. Die DOBON-Gesamtausbeute beträgt 2780 g.

Beispiel 3

Herstellung von Octadecyltrimethylammonium-β-hydroxynaphthoat (OBON)

Ausgangslösungen sind eine wäßrige alkalische Lösung von Octadecyltrimethylammoniumchlorid und eine 2-Naphthol-3-carbonsäure-Lösung in Methylisobutylketon (MIBK).

1830 g Octadecyltrimethylammoniumchlorid werden in 9150 g $H_2O$ bei 80° C gelöst und auf dieser Temperatur gehalten. Nun setzt man unter kräftigem Rühren eine 6 n Natronlauge zu, die vorher aus 216 g Ätznatron und 890 g Wasser hergestellt wurde. Diese alkalische Lösung muß ebenfalls auf 80° C gehalten werden.

1000 g 2-Naphthol-3-carbonsäure (99 %ig) werden in 6600 g MIBK bei 80 - 85° C unter Rühren vollständig in Lösung gebracht. In die 80° C heiße Carbonsäure-Lösung wird nun unter kräftigem Rühren die gesamte alkalische Lösung des quaternären Ammoniumchlorids geschüttet. Das Reaktionsgemisch bleibt bei etwa 80° C weitere 20 Minuten unter kräftiger Rührung. Dann wartet man ebenfalls bei 70 - 80° C die vollständige Phasentrennung ab (klare Phasen).

Die wäßrige NaCl-haltige Phase wird verworfen. Die MIBK-Phase, die 99 % des gebildeten OBON und 10 % des gebildeten NaCl enthält, muß zur Entfernung des NaCl-Restes einer Wasserwäsche unterworfen werden. Um Ausfällungen zu verhindern, wird die Lösung auf Temperaturen zwischen 70° C und 80° C gehalten.

Zur Wasserwäsche verwendet man wegen der besseren Koaleszenzwirkung eine 0,5 %ige $NaHCO_3$-Lösung. Die extraktive Wäsche wird in einem 6-stufigen Extraktor bei 80° C und einem Volumenverhältnis Speiselösung/Waschflüssigkeit= 2/1 ausgeführt. Der OBON-Verlust im Waschwasser beträgt 1 Gew.-%. Der NaCl-Gehalt in der MIBK-Phase wird bei der Wäsche um über 97,8 % von 3,33 mg NaCl/g auf 0,073 mg/g reduziert. Zur Gewinnung des OBON aus der MIBK-Lösung wird das MIBK im Vakuumverdampfer bei Temperaturen unter 60° C destillativ abgetrennt. Die OBON-Gesamtausbeute beträgt 2588 g.

Beispiel 4

Herstellung von Hexadecyltrimethylammonium-heptan-sulfonat (HAHS)

304 g Natrium-heptan-1-sulfonat werden in 985 g Wasser gelöst und in die Lösung 1620 g einer wäßrigen Hexadecyltrimethylammoniumchlorid-Lösung (29,11 %ig) zugesetzt. Dann gibt man 1500 ml Methylisobutylketon (MIBK) hinzu und erwärmt unter kräftiger Rührung 20 min auf 80° C. Nach der Phasentrennung wird die organische (MIBK)-Phase die 96 % des HAHS und 31 % des NaCl enthält bei Raumtemperatur einer Gegenstromwäsche mit einer 0,5 %igen $NaHCO_3$-Lösung (6 Stufen, Phasenvolumenverhältnis org. Phase/Waschlösung= 2/1) unterworfen. Der NaCl-Gehalt in der MIBK-Phase wird bei der Wäsche von 7,42 mg NaCl/g auf 0,091 mg/g reduziert, das sind 98,8 %. Die Gesamtausbeute an HAHS beträgt 643 g.

Beispiel 5

Herstellung von Hexadecyltrimethylammoniumsalicylat (HASA)

360 g Natriumsalicylat werden in 117 g Wasser gelöst und 2466 g einer wäßrigen Hexadecyltrimethylammoniumchlorid-Lösung (29,11 %ig) zugesetzt. Dann gibt man 1500 ml Methylisobutylketon hinzu und erwärmt unter kräftiger Rührung 20 min auf 80° C. Nach der Phasentrennung wird die organische (MIBK)-Phase, die 98 % des HASA und 27 % des NaCl enthält, bei Raumtemperatur einer Gegenstromwäsche, wie im Beispiel 4, unterworfen. Der NaCl-gehalt in der MIBK-Phase wird bei der Wäsche von 6,7 mg/g auf 0,062 mg/g reduziert, das sind 99,1 %. Die Gesamtausbeute an HASA beträgt 918 g.

Beispiel 6

Herstellung von N-Cetylpyridinium-$\beta$-hydroxy-naphthoat (CEBON)

186 g 2-Naphthol-3-carbonsäure ($\beta$-Hydrcxynaphthoesäure) werden bei 80° C in 1280 g Methylisobutylketon gelöst und in diese Carbonsäurelösung bei 80° C eine wäßrige Lösung aus 351 g N-Cetylpyridiniumchlorid und 1040 g Wasser eingegeben. Dann gibt man nach Abkühlung auf 50° C unter kräftigem Rühren langsam 201 g 6n-NaOH hinzu und mischt das System weitere 20 min bei 50° C, bevor man bei dieser Temperatur die Phasen absetzen läßt. Nach der Phasentrennung wird die MIBK-Phase, die 99,2 % des CEBON und 7 % des NaCl enthält, einer Gegenstromwäsche mit einer 1 Gew.-%igen $NaHCO_3$-Lösung unterworfen (Extraktionsbedingungen wie in Beispiel 4). Der NaCl-Gehalt in der organischen (MIBK)-Phase

wird bei der Wäsche von 2,9 mg/g auf 0,071 mg/g reduziert, das sind 97,6 %. Die Gesamtausbeute beträgt 491 g CEBON:

## Ansprüche

1. Verfahren zur Herstellung von quartären Ammoniumsalzen durch Umsetzung von Tetraalkylammonium- oder Alkylpyridinium-Halogeniden mit Alkalisalzen von organischen Carbon- oder Sulfonsäuren, dadurch gekennzeichnet, daß man eine wäßrige Lösung, die Alkalihydroxid und Tetraalkylammonium-oder Alkylpyridinium-Halogenide enthält, vermischt mit einer Lösung von organischen Carbon- oder Sulfonsäuren in einem mit Wasser nicht mischbaren Lösemittel, wobei, man quartäre Ammoniumsalze der Formel

   $R_1$-$K^{\oplus}A^{\ominus}$

   herstellt, wobei $R_1$ $C_{12}$-$C_{26}$-Alkyl oder $C_{12}$-$C_{26}$-Alkenyl, $K^{\oplus}$ eine Gruppe der Formeln

   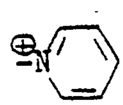

   oder - ${}^{\oplus}N(R_2)_3$, $R_2$ $C_1$-$C_3$-Alkyl, und $A^{\ominus}$ ein Anion der folgenden Formeln bedeutet: $R_3COO^-$ oder $R_3SO_3^{\ominus}$, wo $R_3$ $R_6$-$C_9$-Alkyl oder $C_6$-$C_9$-Alkenyl ist und die Summe der C-Atomen in $R_1$ und $R_3$ mindestens 21 betragen soll;

   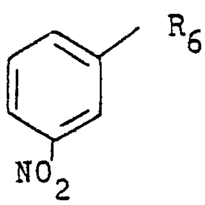 ; 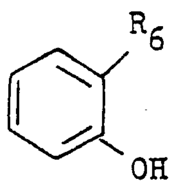 ; 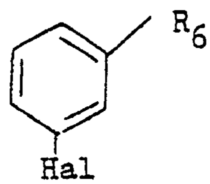 ; 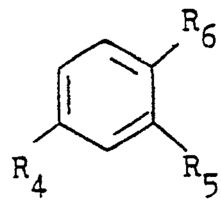 oder

   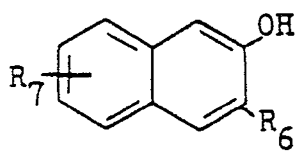

   wo Hal Fluor Chlor, Brom oder Jod $R_4$ $C_1$-$C_5$-Alkyl, $C_2$-$C_5$-Alkenyl oder $C_1$-$C_5$-Alkoxy in den Stellungen 3, 4, 5 und 6, $R_5$ Wasserstoff oder Hydroxy in den Stellungen 2 und 3 $R_6$ $COO^-$ oder $SO_3$ und $R_7$ Wasserstoff oder Methyl ist.

2. Verfahren nach Anspruch 1, dadurch gekennzeichnet, daß man nach der Reaktion aus der das quartäre Ammoniumsalz enthaltenden organischen Phase die Restmengen an Alkalihalogenid mit Wasser ein- oder mehrstufig auswäscht.

3. Verfahren nach Anspruch 1, dadurch gekennzeichnet, daß man als organische Phase Methylisobutylketon verwendet.

4. Verfahren nach Anspruch 1, dadurch gekennzeichnet, daß man die organischen Carbon- und Sulfonsäuren in Form ihrer Alkalisalze einnimmt und auf die Zugabe von Alkalihydroxid verzichtet.

## Claims

1. A process for the preparation of a quaternary ammonium salt by reaction of a tetraalkylammonium or alkylpyridinium halide with an alkali metal salt of an organic carboxylic or sulfonic acid, which comprises mixing an aqueous solution containing an alkali metal hydroxide and a tetraalkylammonium or alkylpyridinium halide with a solution of an organic carboxylic or sulfonic acid in a water-immiscible solvent, wherein a quaternary

ammonium salt of the formula

$R_1\text{-}K^{\oplus}A^{\ominus}$

in which $R_1$ denotes $C_{12}$–$C_{26}$-alkyl or $C_{12}$-$C_{26}$-alkenyl, $K^{\oplus}$ denotes a group of the formula

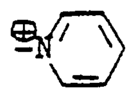

or $-^{\oplus}N(R_2)_3$, $R_2$ denotes $C_1$-$C_3$-alkyl and $A^{\ominus}$ denotes an anion of the following formulae: $R_3COO^-$ or $R_3SO_3^{\ominus}$, where $R_3$ is $C_6$-$C_9$-alkyl or $C_6$-$C_9$-alkenyl and the sum of C atoms in $R_1$ and $R_3$ should be at least 21;

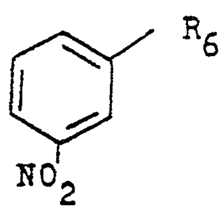 ; 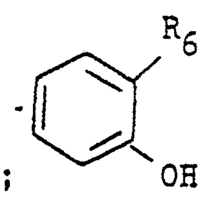 ; 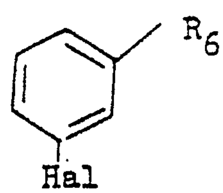 ; 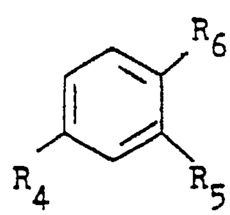 or

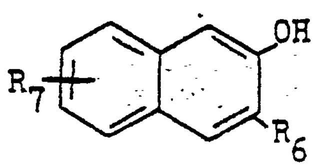

where Hal is fluorine, chlorine, bromine or iodine, $R_4$ is $C_1$-$C_5$-alkyl, $C_2$-$C_5$-alkenyl or $C_1$-$C_5$-alkoxy in positions 3, 4, 5 or 6, $R_5$ is hydrogen or hydroxyl in positions 2 or 3, $R_6$ is $COO^-$ or $SO_3^-$ and $R_7$ is hydrogen or methyl, is prepared

2. The process as claimed in claim 1, wherein, after the reaction, the residual amount of alkali metal halide is washed out of the organic phase containing the quaternary ammonium salt with water in a single- or multi-stage process.

3. The process as claimed in claim 1, wherein methyl isobutyl ketone is used as the organic phase.

4. The process as claimed in claim 1, wherein the organic carboxylic or sulfonic acid is taken in the form of one of its alkali metal salts and the addition of an alkali metal hydroxide is dispensed with.

## Revendications

1. Procédé pour préparer des sels d'ammoniums quaternaires par réaction d'halogénures de tétraalkylammoniums ou d'alkyl-pyridiniums avec des sels de métaux alcalins d'acides carboxyliques ou sulfoniques organiques, procédé caractérisé en ce qu'on mélange une solution aqueuse qui contient un hydroxyde de métal alcalin et des halogénures de tétraalkyl-ammoniums ou d'alkyl-pyridiniums, avec une solution d'acides carboxyliques ou sulfoniques organiques dans un solvant non miscible à l'eau, et

on prépare ainsi des sels d'ammoniums quaternaires répondant à la formule :

$$R_1-K^{\oplus}\ A^{\ominus} \qquad (I)$$

dans laquelle

R1 représente un alkyle en $C_{12}$-$C_{26}$ ou un alcényle en $C_{12}$-$C_{26}$,

$K^{\oplus}$ représente un radical répondant à l'une des formules :

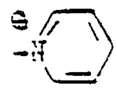

et - $^{\oplus}N(R_2)_3$ les symboles $R_2$ de cette dernière représentant chacun un alkyle en $C_1$-C3, et

$A^{\ominus}$ représente un anion répondant à l'une des formules suivantes :

$R_3COO^{\ominus}$ ; $R_3SO_3^{\ominus}$

où $R_3$ représente un alkyle en $C_6$-$C_9$ ou un alcényle en $C_6$ -$C_9$, la somme du nombre des atomes de carbone de $R_1$ et de $R_3$ devant être au moins égale à 21

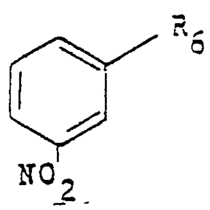 ; 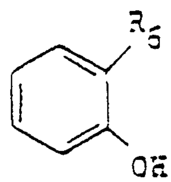 ; 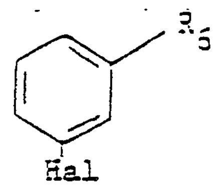 ;

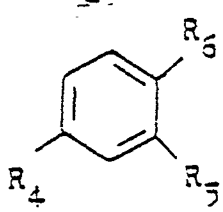 et 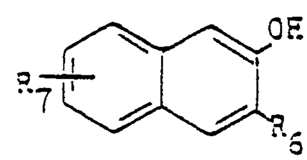

; où Hal représente le fluor, le chlore, le brome ou l'iode, $R_4$ représente un alkyle en $C_1$-$C_5$, un alcényle en $C_2$-$C_5$ ou un alcoxy en $C_1$-$C_5$ en l'une des positions 3, 4, 5 et 6, $R_5$ représente l'hydrogène ou un hydroxy en l'une des positions 2 et 3, $R_6$ représente $-COO^-$ ou $-SO_3^-$,et $R_7$ représente l'hydrogène ou un méthyle.

2. Procédé selon la revendication 1 caractérisé en ce qu'après la réaction on extrait les quantités résiduelles de l'halogénure de métal alcalin par lavage à l'eau, en un ou plusieurs étages, à partir de la phase organique contenant le sel d'ammonium quaternaire.

3. Procédé selon la revendication 1 caractérisé en ce qu'on utilise la méthyl-isobutyl-cétone comme phase organique.

4. Procédé selon la revendication 1 caractérisé en ce qu'on met en jeu les acides carboxyliques et sulfoniques organiques sous la forme de leurs sels de métaux alcalins et on n'ajoute pas d'hydroxyde de métal alcalin.